# EUROPÄISCHE PATENTANMELDUNG

(11) **EP 0 021 113 A1**
(43) Veröffentlichungstag der Anmeldung: **07.01.1981**
(21) Anmeldenummer: 80103021.4
(22) Anmeldetag: 30.05.1980
(51) Int. Cl.: C07C 69/74, C07C 69/63, C07C 51/60, C07C 55/40

(54) **Verfahren zur Herstellung von 3,3-Dimethyl-cyclopropan-1,2-dicarbonsäureestern, Zwischenprodukte hierfür und deren Herstellung**

(30) Priorität: 12.06.1979 DE 2923776
(71) Anmelder: BAYER AG, 51368 Leverkusen (DE)
(72) Erfinder: Maurer, Fritz, Dr., D-5600 Wuppertal 1 (DE); Riebel, Hans-Jochem, Dr., D-5600 Wuppertal 1 (DE); Priesnitz, Uwe, Dr., D-4750 Unna-Massen (DE)

(57) **Zusammenfassung**

Gegenstand der vorliegenden Erfindung sind ein neues Verfahren zur Herstellung von 3,3-Dimethyl-cyclo- propan-1,2-dicarbonsäureestern der Formel 1 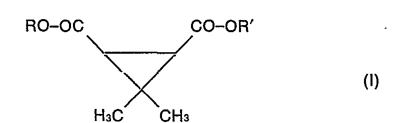 in welcher
R und R' die in der Beschreibung angegebene Bedeutung haben, das dadurch gekennzeichnet ist, daß man 1,3-Dichlor-2,2-dimethyl-propan-1,3-dicarbonsäureester der Formel 11 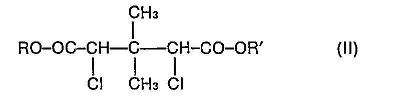 mit Zink, gegebenenfalls in Gegenwart eines Verdünnungsmittels, bei Temperaturen zwischen 20 und 200° C umsetzt und neue 1,3-Dichlor-2,2-dimethyl-propan-1,3-di- carbonsäureester der Formel 11 sowie Verfahren und Zwischenprodukte zu ihrer Herstellung.

## Beschreibung

Die Erfindung betrifft ein neues Verfahren zur Herstellung von bekannten 3,3-Dimethyl-cyclopropan-1,2-dicarbonsäureestern sowie Zwischenprodukte zu ihrer Herstellung und ein neues Verfahren zur Herstellung dieser Zwischenprodukte.

Es ist bereits bekannt geworden, daß man cis- und/oder trans-3,3-Dimethyl-cyclopropan-1,2-dicarbonsäure-dimethylester erhält, wenn man Maleinsäure- und/oder Fumarsäure-dimethylester mit Diphenylsulfonium-isopropylid bei -7o°C umsetzt (vergleiche J.Am. Chem.Soc. 89 (1967), 3912 - 3914; ibid. 95 (1973), 3970 - 3976).

Diphenylsulfonium-isopropylid wird durch Umsetzung von Diphenyl-äthyl-sulfonium-tetrafluorborat mit Lithium-diisopropylamid, anschließende Alkylierung mit Methyliodid und erneute Umsetzung mit Lithium-diisopropylamid bei -7o°C hergestellt.

An Stelle von Diphenylsulfonium-isopropylid kann auch Triphenylphosphonium-isopropylid zur Synthese von 3,3-Dimethyl-cyclopropan-1,2-dicarbonsäureestern ausgehend von Maleinsäure- und/oder Fumarsäureestem verwendet werden (vergleiche Tetrahedron Lett. 1978, 1847-1850).

Die Umsetzungen mit den genannten Yliden werden wegen deren Empfindlichkeit gegen Luftsauerstoff und Feuchtigkeit unter einer Inertgasatmosphäre und unter Verwendung von sorgfältig getrockneten Lösungsmitteln durchgeführt; das gleiche gilt auch für die Herstellung der Ylide; hierfür werden starke Basen, wie z.B. Butyllithium und/oder Lithium-diisopropylamid, verwendet.

Die bekannten Synthesemethoden für 3,3-Dimethyl-cyclo- propan-1,2-dicarbonsäureester, welche unter Verwendung der oben genannten Ylide durchgeführt werden, dürften wegen des hohen Aufwandes - auch bei der Herstellung von Vorprodukten - für die Herstellung von 3,3-Dimethyl- cyclopropan-1,2-dicarbonsäureestern im industriellen Bereich wenig geeignet sein.

Wegen der möglichen Bedeutung von 3,3-Dimethyl-cyclo- propan-1,2-dicarbonsäure-derivaten als Zwischenprodukte für die Herstellung von Pyrethroiden bestand nun Interesse an einem einfacheren und kostengünstigeren Verfahren, nach welchem diese Verbindungen in guten Ausbeuten und in hoher Reinheit herzustellen sein sollten.

Zur Herstellung von 3,3-Dimethyl-cyclopropan-1,2-dicarbonsäure ist auch ein Verfahren bekannt geworden, bei welchem diese Verbindung durch Umsetzung von 1-Brom-2,2-dimethyl-3-äthoxycarbonyl-propan-carbonsäure mit Kaliumcarbonat in Alkohol und anschließendes Behandeln des Reaktionsgemisches mit wäßriger Salzsäure erhalten wird (vergleiche J.Chem.Soc. (London) 75 (1899), 48-61. Nach diesem Verfahren erhält man 3,3-Dimethyl-cyclo- propan-1,2-dicarbonsäure jedoch nur in unbefriedigenden Ausbeuten; zudem kann das Ausgangsprodukt nach dem Stand der Technik nur in geringer Ausbeute erhalten werden und ist außerdem durch Nebenprodukte stark verunreinigt.

Gegenstand der vorliegenden Erfindung sind
(1) ein neues Verfahren zur Herstellung von 3,3-Dimethyl- cyclopropan-1,2-dicarbonsäureestern der Formel I 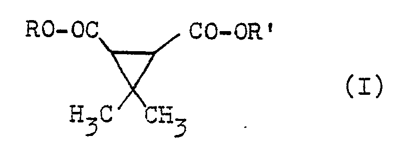 in welcher
   R und R' gleich oder verschieden sind und für Alkyl stehen, dadurch gekennzeichnet, daß man 1,3-Dichlor-2,2-dimethyl-propan-1,3-dicarbonsäureester der Formel II 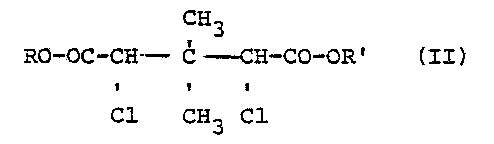 in welcher
   R und R' gleich oder verschieden sind und für Alkyl stehen,
   mit Zink, gegebenenfalls in Gegenwart eines Verdünnungsmittels, bei Temperaturen zwischen 2o und 2oo°C umsetzt;
(2) neue 1,3-Dichlor-2,2-dimethyl-propan-1,3-dicarbon- säureester der Formel II 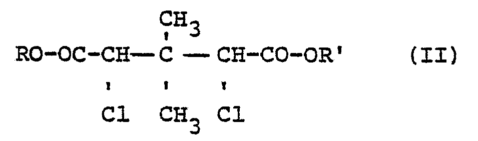 in welcher
   R und R' gleich oder verschieden sind und für Alkyl stehen;
(3) ein Verfahren zur Herstellung von 1,3-Dichlor-2,2-dimethyl-propan-1,3-dicarbonsäureestern der Formel II (oben), dadurch gekennzeichnet, daß man 2,2-Dimethyl-propan-1,3-dicarbonsäure-derivate der Formel III 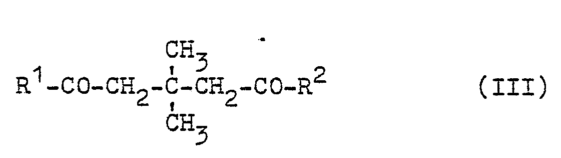 in welcher
   R¹ für Hydroxy oder Alkoxy steht und
   R² für Hydroxy oder zusammen mit R¹ für ein Anhydrid-Sauerstoffatom steht,

   mit einem Säurechlorid, gegebenenfalls in Gegenwart eines Katalysators, und mit einem Chlorierungsmittel bei Temperaturen zwischen 0 und 15o°C umsetzt und die auf diese Weise gebildeten 1,3-Dichlor-2,2-dimethylpropan-1,3-dicarbonsäure-derivate der Formel IV 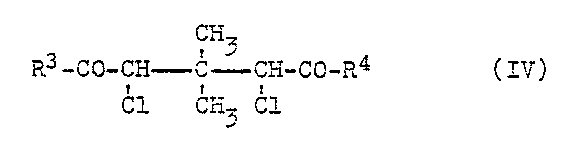 in welcher
   ^{R3} für Chlor oder Alkoxy steht und
   R⁴ für Chlor oder zusammen mit R³ für ein Anhydrid-Sauerstoffatom steht,

   durch Umsetzung mit Alkoholen nach üblichen Methoden verestert;
(4) neue 1,3-Dichlor-2,2-dimethyl-propan-1,3-dicarbonsäure-derivate der Formel IV 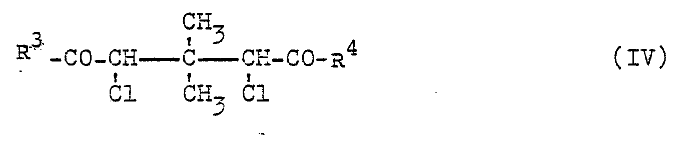 in welcher
   _{R}³ für Chlor oder Alkoxy steht und
   R⁴ für Chlor oder zusammen mit R³ für ein Anhydrid-Sauerstoffatom steht.

Überraschenderweise können nach dem erfindungsgemäßen Verfahren (1), verbunden mit dem unter (3) beschriebenen Verfahren zur Herstellung neuer Zwischenprodukte, 3,3-Dimethyl-cyclopropan-1,2-dicarbonsäureester der Formel (I) wesentlich einfacher und kostengünstiger als nach bekannten Methoden in sehr guten Ausbeuten und in hoher Reinheit hergestellt werden. Das neue Verfahren ist daher eher als die bisher bekannten Verfahren zur industriellen Herstellung von 3,3-Dimethyl-cyclopropan-1,2-dicarbonsäureestern geeignet.

Verwendet man bei Verfahren (3) als Ausgangsverbindung beispielsweise 3,3-Dimethyl-propan-1,3-dicarbonsäureanhydrid sowie als Chlorierungsmittel Sulfurylchlorid, und als Veresterungsmittel Methanol, so kann der Reaktionsablauf bei Verfahren (3) und die Reaktion des dabei erhaltenen 1,3-Dichlor-2,2-dimethyl-propan-1,3-dicarbonsäure-dimethylesters mit Zink in Verfahren (1) durch folgendes Formelschema skizziert werden:

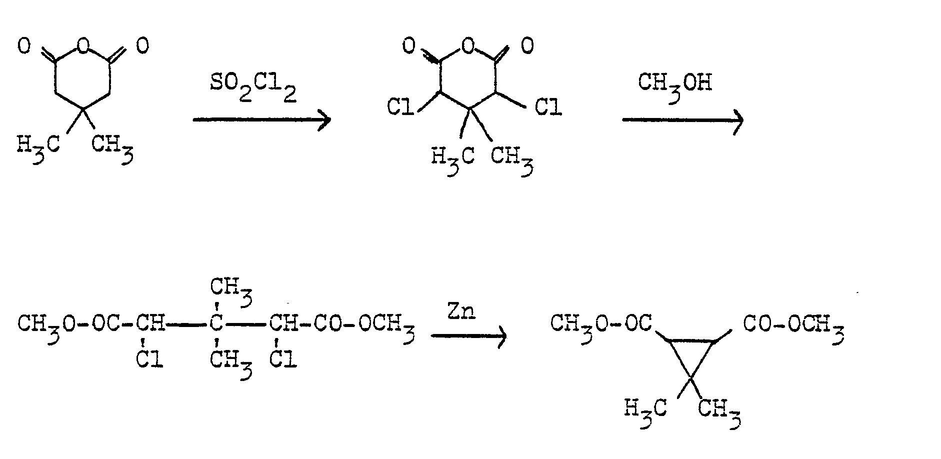

Die bei Verfahren (1) als Ausgangsverbindungen zu verwendenden neuen 1,3-Dichlor-2,2-dimethyl-propan-1,3-dicarbonsäureester sind durch Formel (II) definiert. Vorzugsweise steht darin
R für C₁-C₄-Alkyl, insbesondere für Methyl oder Äthyl.

Als Beispiele seien genannt :
1,3-Dichlor-2,2-dimethyl-propan-1,3-dicarbonsäure- dimethylester, -diäthylester, -di-n-propylester, -di-iso-propylester, -di-n-butylester, -di-iso-butylester, -di-sek.-butylester, -di-tert.-butylester, -methyl- äthylester,-methyl-propylester, -methyl-isopropylester und -methyl-n-butylester.

Man erhält die neuen 1,3-Dichlor-2,2-dimethyl-propan-1,3-dicarbonsäureester nach dem oben unter (3) beschriebenen Verfahren durch Umsetzung von 2,2-Dimethyl-propan-1,3-dicarbonsäure-derivaten der Formel III (oben) mit einem Säurechlorid, gegebenenfalls in Gegenwart eines Katalysators, und mit einem Chlorierungsmittel, welches gegebenenfalls mit dem Säurechlorid identisch ist, bei Temperaturen zwischen 0 und 15o°C.

Als Säurechloride, welche bei Verfahren (3) zu verwenden sind, seien beispielsweise Phosphorpentachlorid, Phosphoroxychlorid, Phosphortrichlorid, Thionylchlorid, Sulfurylchlorid und Phosgen genannt. Vorzugsweise werden Phosphorpentachlorid, Thionylchlorid und Sulfurylchlorid verwendet.

Verfahren (3) wird gegebenenfalls unter Verwendung von Katalysatoren durchgeführt. Als solche kommen Verbindungen in Betracht, welche gewöhnlich bei der Herstellung von Carbonsäurechloriden als Katalysatoren eingesetzt werden. Hierzu gehören insbesondere tertiäre und heterocyclische Amine wie z.B. Triäthylamin, N,N-Dimethyl-anilin und Pyridin sowie Ammoniumsalze wie z.B. Tetraäthylammoniumchlorid, Carbonsäureamide wie z.B. Dimethylformamid, Dimethylacetamid und N-Methyl-pyrrolidon, ferner Phosphorverbindungen wie z.B. Triphenylphosphin, Triphenylphosphinoxid oder Hexamethyl-phosphorsäuretriamid.

Als Chlorierungsmittel, welche bei Verfahren (3) eingesetzt werden können, seien beispielsweise Sulfurylchlorid und elementares Chlor genannt.

In einer bevorzugten Variante (a) von Verfahren (3) wird 2,2-Dimethyl-propan-1,3-dicarbonsäure-anhydrid (β,β-Dimethylglutarsäureanhydrid) mit etwa der äquimolaren Menge (o,9 bis 1,2 Mol) Phosphorpentachlorid auf eine Temperatur zwischen 8₀ und 14o°C erhitzt und bei dieser Temperatur werden etwa 2 Moläquivalente (1,9 bis 2,2 Mol) Chlor in die Mischung eingeleitet oder 2 bis 3 Moläquivalente Sulfurylchlorid zugetropft.

In einer zweiten bevorzugten Variante (b) von Verfahren (3) wird 2,2-Dimethyl-propan-1,3-dicarbonsäure-anhydrid bei Temperaturen zwischen 11o und 15o°C mit überschüssigem Sulfurylchlorid (3 bis 5 Mol je Mol Anhydrid) tropfenweise versetzt.

In einer dritten bevorzugten Variante (c) von Verfahren (3) wird ein 2,2-Dimethyl-propan-1,3-dicarbonsäure- ester(mono-ester) (ß,ß-Dimethylglutarsäure-monoester) mit etwa 1,2 bis 3 Moläquivalenten Thionylchlorid, gegebenenfalls in Gegenwart eines der oben angegebenen Katalysatoren, auf eine Temperatur zwischen 4o und 9o°C erwärmt und bei etwa 8o°C mit etwa 2 Moläquivalenten (1,9 bis 2,2 Mol je Mol Ester) Sulfurylchlorid tropfenweise versetzt.

In einer vierten bevorzugten Variante (d) von Verfahren (3) wird 2,2-Dimethyl-propan-l,3-dicarbonsäure (ß,ß-Di- methylglutarsäure) mit etwa 2 Moläquivalenten (1,9 bis 2,2 Mol je Mol Säure) Phosphorpentachlorid bei Temperaturen zwischen lo und 5o°C umgesetzt. In das Reaktionsgemisch werden bei einer Temperatur zwischen 8₀ und 14o°C etwa 2 Moläquivalente (1,9 bis 2,2 Mol) Chlor eingeleitet und die Mischung wird einige Stunden bei 7o bis 9o°C gerührt.

Die nach den Verfahrensvarianten (3 a) bis (3 d) erhaltenen 1,3-Dichlor-2,2-dimethyl-propan-1,3-dicarbonsäure-derivate der Formel (IV) können durch Destillation oder durch sorgfältiges Abziehen flüchtiger Komponenten bei mäßig erhöhter Temperatur und stark vermindertem Druck (sogenanntes "Andestillieren" gereinigt werden. Die Zwischenprodukte der Formel (IV) können jedoch auch ohne Zwischenisolierung, gegebenenfalls nach Abdestillieren überschüssiger Halogenierungsmittel, nach üblichen Methoden durch Umsetzung mit Alkoholen bei Temperaturen zwischen lo und 3o°C verestert werden.

Zur Reinigung und Isolierung der Ester der Formel (II) wird entweder eingeengt und der Rückstand in einem mit Wasser nicht mischbaren Lösungsmittel wie z.B. Äther aufgenommen, oder das Veresterungsgemisch wird mit Wasser verdünnt und mit einem mit Wasser nicht mischbaren Lösungsmittel, wie z.B. Äther oder Toluol extrahiert. Die organische Phase wird neutral gewaschen, getrocknet und eingeengt. Die im Rückstand verbleibenden Produkte können durch Vakuumdestillation gereinigt werden.

Die bei Verfahren (3) anfallenden neuen 1,3-Dichlor-2,2-dimethyl-propan-1,3-dicarbonsäure-derivate sind durch Formel (IV) definiert. Vorzugsweise stehen darin
_{R}³ für Chlor oder C₁-C₄-Alkoxy, insbesondere Methoxy oder Äthoxy, und
_{R}⁴ für Chlor oder zusammen mit R3 für ein Anhydrid-Sauerstoffatom.

Als Beispiele seien genannt:
1,3-Dichlor-2,2-dimethyl-propan-1,3-dicarbonsäure- dichlorid, 1,3-Dichlor-2,2-dimethyl-propan-1,3-dicarbonsäure-anhydrid, 1,3-Dichlor-2,2-dimethyl-propan-1,3-dicarbonsäure-chlorid- -methylester, -äthylester, -n-propylester, -iso-propylester, n-butylester, -iso-butylester, -sek.-butylester und -tert.-butylester.

Die bei Verfahren (3) als Ausgangsstoffe einzusetzenden 2,2-Dimethyl-propan-l,3-dicarbonsäure-derivate sind durch Formel (III) definiert. Vorzugsweise stehen darin
R für Hydroxy oder C₁-C₄-Alkoxy, insbesondere Methoxy oder Äthoxy, und
R² für Hydroxy oder zusammen mit R¹ für ein Anhydrid-Sauerstoffatom.

Als Beispiele seien genannt:
2,2-Dimethyl-propan-l,3-dicarbonsäure, 2,2-Dimethylpropan-1,3-dicarbonsäure-anhydrid, 2,2-Dimethyl-propan-1,3-dicarbonsäure- -monomethylester, -monoäthylester, -mono-n-propylester, -mono-iso-propylester, -mono-n-butylester, -mono-iso-butylester, -mono-sek.-butylester und -mono-tert.-butylester.
2,2-Dimethyl-propan-1,3-dicarbonsäure-derivate der Formel (III) sind bekannt (vergleiche J.Org.Chem. 15 (1950), 85o und 855).

Das erfindungsgemäße Verfahren (1) wird vorzugsweise unter Verwendung von Verdünnungsmitteln durchgeführt. Als solche kommen insbesondere aprotisch-polare Solventien in Betracht. Hierzu gehören insbesondere Carbonsäureamide wie z.B. Dimethylformamid, Dimethylacetamid und N-Methyl-pyrrolidon, Sulfoxide und Sulfone wie z.B. Dimethylsulfoxid und Tetramethylensulfon, Phosphorsäureamide wie z.B. Hexamethyl-phosphorsäuretriamid und Äther wie z.B. Glycol-dimethyläther, Diglycol-dimethyläther, Tetrahydrofuran und Dioxan sowie Nitrile wie z.B. Acetonitril und Propionitril.

Die Reaktionstemperatur liegt beim erfindungsgemäßen Verfahren (1) im allgemeinen zwischen 2o und 2oo°C, vorzugsweise zwischen 8o und 15o°C. Die Umsetzung wird im allgemeinen bei Normaldruck oder einem dem Dampfdruck des Verdünnungsmittels angepaßten Druck durchgeführt.

Zur Durchführung des erfindungsgemäßen Verfahrens (1) setzt man auf 1 Mol 1,3-Dichlor-2,2-dimethyl-propan-1,3-dicarbonsäureester (II) zwischen 1 und 2,5 Mol, vorzugsweise zwischen 1,5 und 2,2 Mol Zink ein.

In einer bevorzugten Ausführungsform des erfindungsgemäßen Verfahrens (1) wird das Zink in einem der oben angegebenen Verdünnungsmittel auf die erforderliche Reaktionstemperatur, vorzugsweise auf 9o bis 11o°C aufgeheizt und tropfenweise mit einem Ester der Formel (II) versetzt. Das Reaktionsgemisch wird nach Abklingen der exothermen Reaktion noch einige Stunden bei 1oo bis 12o°C gerührt, abgekühlt und abgesaugt. Zur Reinigung und Isolierung des Reaktionsproduktes wird das Filtrat mit einem mit wasser nicht mischbaren Lösungsmittel, wie z.B. Toluol, versetzt, mit verdünnter Salzsäure und dann mit Wasser gewaschen, getrocknet und eingeengt. Das Reaktionsprodukt kann durch Vakuumdestillation gereinigt werden. Zur Charakterisierung dient der Siedepunkt.

Die nach dem erfindungsgemäßen Verfahren herzustellenden 3,3-Dimethyl-cyclopropan-1,2-dicarbonsäureester können als Zwischenprodukte zur Herstellung von insektizid und akarizid wirksamen Pyrethroiden verwendet werden (vergleiche Pestic. Sci. 7 (1976), 492-498; Tetrahedron Lett. 1978, 1847-185₀).

### Herstellungsbeispiele

### Beispiel 1:

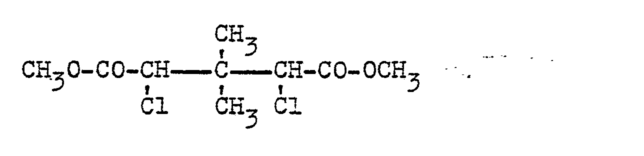

Eine Mischung von 28,4 g (o,2 Mol) 3,ß-Dimethylglutarsäureanhydrid und 41,6 g (o,2 Mol) Phosphorpentachlorid wird eine halbe Stunde auf 115-12o°C erwärmt. Dann werden bei 115-12o°C 28 g (o,4 Mol) Chlor eingeleitet und es wird auf 2o°C abkühlen gelassen. Zu dieser Mischung werden nun bei 2o°C 3oo ml Methanol getropft, und es wird 12 Stunden bei 2o°C nachgerührt. Anschließend wird das Methanol abgezogen. Der Rückstand wird in 3oo ml Äther aufgenommen. Die Ätherlosung wird einmal mit 2oo ml 2%iger Natriumcarbonatlösung und 2 mal mit je 2oo ml Wasser gewaschen, dann über Natriumsulfat getrocknet und eingeengt. Der Rückstand wird destilliert. Man erhält 48 g (93,5% der Theorie) 1,3-Dichlor-2,2-dimethyl-propan-1,3-dicarbonsäure-dimethylester in Form eines farblosen Öls vom Siedepunkt 99-1o2°C/l mm Hg.

### Beispiel 2:

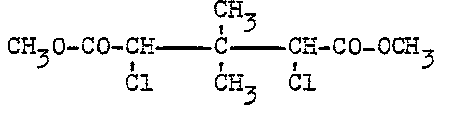

Eine Mischung von 28,4 g (o,2 Mol) ß,ß-Dimethylglutarsäureanhydrid und 41,6 g (o,2 Mol) Phosphorpentachlorid wird eine halbe Stunde auf 125-13o°C erhitzt. Dann werden zu dieser Reaktionsmischung 68 g (o,5 Mol) Sulfurylchlorid bei einer Temperatur von 125°C zugetropft. Nachdem die Sulfurylchloridzugabe beendet ist, wird eine Stunde bei 13o°C nachgerührt und dann auf 2o°C abkühlen gelassen. Jetzt werden 3oo ml Methanol in das Reaktionsgemisch getropft und es wird 12 Stunden bei 2o°C nachgerührt. Dann wird das Methanol abgezogen und der Rückstand mit 3oo ml Äther versetzt. Die Ätherlösung wird einmal mit 3oo ml 2%igem Natriumbicarbonat und zweimal mit je 2oo ml Wasser gewaschen und dann eingeengt. Der Rückstand wird destilliert. Man erhält 42 g (82% der Theorie) 1,3-Dichlor-2,2-dimethyl-propan-1,3-dicarbonsäure-dimethylester in Form eines farblosen Öles vom Siedepunkt 1oo-1o2°C/2 mm Hg.

### Bei-spiel- 3:

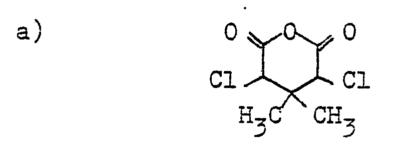

Zu einer Schmelze von 28,4 g (o,2 Mol) β,β-Dimethylglutarsäureanhydrid werden bei 125-13o°C 11o g (o,3 Mol) Sulfurylchlorid in der Art getropft, daß die Reaktionstemperatur immer zwischen 125 und 13o°C liegt (der große Sulfurylchloridüberschuß ist notwendig, da bei der hohen Reaktionstemperatur Verluste an Sulfurylchlorid durch Verdampfen entstehen). Nachdem die Sulfurylchloridzugabe beendet ist, wird noch lo Minuten bei 13o°C nachgerührt. Dann wird andestilliert. Man erhält 4o g 1,3-Dichlor-2,2-dimethyl-propan-1,3-dicarbonsäureanhydrid, das direkt weiterverarbeitet wird.

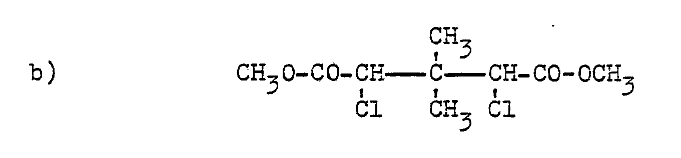

Zu 4o g (o,19 Mol) des nach Verfahren ₃ a) hergestellten 1,3-Dichlor-2,2-dimethyl-propan-1,3-dicarbonsäureanhydrids werden bei 25°C 3oo ml Methanol und 2 ml konzentrierter Schwefelsäure getropft. Dann wird 2 Stunden unter Rückfluß erhitzt. Anschließend wird wie in Beispiel 1 beschrieben aufgearbeitet. Man erhält 42 g (82% der Theorie) bezogen auf β,β-Dichlor-2,2-dimethylpropan-l,3-dicarbonsäure-dimethylester in Form eines farblosen Öles vom Siedepunkt 99-1o4°C/2 mm Hg.

### Beispiel 4:

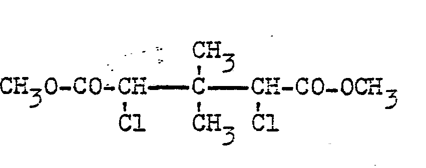

87 g (o,5 Mol) β,β-Dimethyl-glutarsäuremonomethylester (Darstellung s. S.F.Birch et al., J.Chem.Soc.(London ) 1952, 1364) werden zu 15o ml Thionylchlorid getropft und dann 1 Stunde unter Rückfluß gekocht. Unter weiterem Sieden tropft man dann innerhalb von etwa 3 Stunden 148,6 g (1,1 Mol) Sulfurylchlorid zu und kocht nach Ende der Zugabe noch 2 Stunden nach. Das Reaktionsgemisch wird im Vakuum bei ca. 5o°C von überschüssigem Thionylchlorid befreit und dann zu 25o ml Methanol getropft, wobei die Temperatur bis zum Siedepunkt des Methanols ansteigt. Man rührt 1 Stunde nach und destilliert dann das Lösungsmittel im Vakuum ab. Der Rückstand wird in 4oo ml Toluol gelöst und 1 mal mit Natriumbicarbonatlösung und 2 mal mit Wasser gewaschen. Die organische Phase wird über Natriumsulfat getrocknet und im Vakuum eingeengt. Den Rückstand destilliert man im Hochvakuum. Man erhält so 94 g (73% der Theorie) 1,3-Dichlor-2,2-dimethyl-propan-1,3-dicarbonsäure- dimethylester als farbloses Öl mit dem Siedepunkt 8o-85°C/o,o5 mm Hg.

### Beispiel 5:

### 1. Stufe:

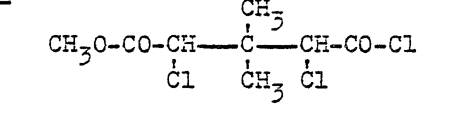

87 g (o,5 Mol) β,β-Dimethyl-glutarsäuremonomethylester (Darstellung s. S.F.Birch et al., J. Chem.Soc.(London) 1952, 1364) werden zu 15o ml Thionylchlorid getröpft und dann 1 Stunde unter Rückfluß gekocht. Unter weiterem Sieden tropft man dann innerhalb von etwa 3 Stunden 148,6 g (1,1 Mol) Sulfurylchlorid zu und kocht nach Ende der Zugabe noch 2 Stunden nach. Das Reaktionsgemisch wird im Vakuum bei ca. 5o°C von überschüssigem Thionylchlorid befreit und dann im Vakuum destilliert. Man erhält auf diese weise lo4 g (8o% der Theorie) 1,3-Dichlor-2,2-dimethyl-propan-1,3-dicarbonsäure- methylesterchlorid in Form eines farblosen Öles mit dem Siedepunkt 128-132°C/1o mm Hg.

### 2. Stufe:

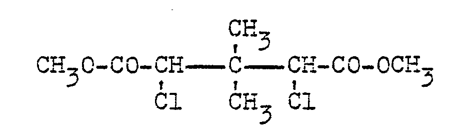

Ein Gemisch aus 65,4 g (o,25 Mol) des oben beschriebenen Esterchlorides und 15o ml Methanol wird 2 Stunden bei Raumtemperatur gerührt, auf 5oo ml Wasser gegossen, und 3 mal mit je loo ml Toluol extrahiert. Die organische Phase wird erst mit 5o ml gesättigter Natriumhydrogencarbonatlösung und dann mit 5o ml Wasser gewaschen, über Natriumsulfat getrocknet und im Vakuum eingedampft. Der Rückstand wird im Vakuum destilliert. Man erhält so 41,6 g (81% der Theorie) 1,3-Dichlor-2,2-dimethyl-pronan-1,3-dicarbonsäuredimethylester mit dem Siedepunkt 134-138⁰C/lo mm Hg.

### Beispiel 6:

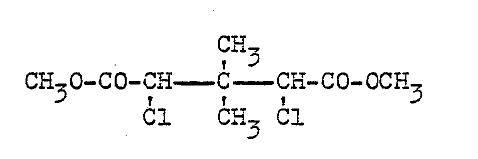

Zu 131 g (o,63 Mol) Phosphorpentachlorid gibt man portionsweise 48,1 g (o,3 Mol) β,β-Dimethyl-glutarsäure so zu, daß die Temperatur nicht über 5o°C steigt. In das so erhaltene Gemisch wird bei 11o°C innerhalb von 3 Stunden 44,7 g (o,63 Mol) Chlor eingeleitet und 2 Stunden bei 8o°C nachgerührt. Nach Abdestillieren der flüchtigen Anteile tropft man das Produkt zu 15o ml Methanol und rührt 1 Stunde nach. Das Reaktionsgemisch wird mit 3oo ml Wasser versetzt und zweimal mit je 25o ml Toluol ausgeschüttelt. Die organischen Phasen werden erst mit 1oo ml gesättigter Natriumhydrogencarbonatlösung und dann.zweimal mit je loo ml Wasser gewaschen, über Natriumsulfat getrocknet und im Vakuum eingedampft. Den Rückstand destilliert man im Vakuum. Man erhält so 6₀,7 g (79% der Theorie) 1,3-Dichlor-2,2-dimethyl-propan-l,3-dicarbonsäuredimethylester mit dem Siedepunkt 128-131°C/7 mm Hg.

### Beispiel 7:

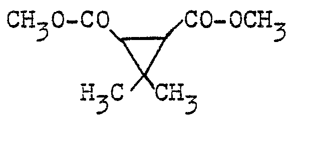

Zu einer Suspension von 13 g (o,2 g-Atom) Zinkstaub in 6o ml Dimethylformamid tropft man bei ca. loo°C 25,6 g (o,1 Mol) 1,3-Dichlor-2,2-dimethyl-propan-1,3-dicarbonsäuredimethylester. Bei der Zugabe steigt die Temperatur des Reaktionsgemisches auf ca. 12o°C an. Nach Ende der exothermen Reaktion hält man die Temperatur noch 2 Stunden bei ca. 1oo°C und kühlt dann auf Raumtemperatur ab. Das Gemisch wird mit 2oo ml Toluol versetzt und vom anorganischen Material abgesaugt. Das Filtrat schüttelt man einmal mit 5o ml lo%iger Salzsäure und dann zweimal mit je loo ml Wasser aus, trocknet es über Natriumsulfat und destilliert das Lösungsmittel im Vakuum ab. Durch Vakuumdestillation des Rückstandes erhält man 13,6 g (73% der Theorie) 3,3-Dimethyl-cyclopropan-1,2-dicarbonsäuredimethylester in Form einer farblosen Flüssigkeit mit dem Siedepunkt 1o2-1o6°C/1o mm Hg.

## Patentansprüche

1) Verfahren zur Herstellung von 3,3-Dimethylcyclo- propan-1,2-dicarbonsäureestern der Formel I 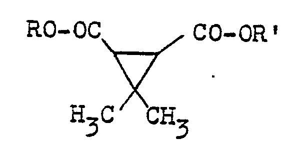 in welcher
R und R' gleich oder verschieden sind und für Alkyl stehen,
dadurch gekennzeichnet, daß man 1,3-Dichlor-2,2-dimethyl-propan-1,3-dicarbonsäureester der Formel II 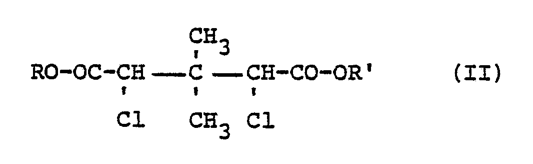 in welcher
R und R' gleich oder verschieden sind und für Alkyl stehen,
mit Zink, gegebenenfalls in Gegenwart eines Verdünnungsmittels, bei Temperaturen zwischen 2o und 2oo°C umsetzt.

2. 1,3-Dichlor-2,2-dimethyl-propan-1,3-dicarbonsäure- ester der Formel II 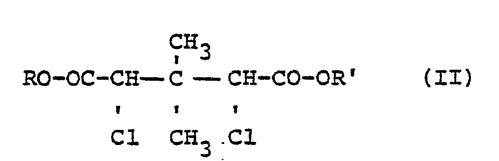 in welcher
R und R' gleich oder verschieden sind und für Alkyl stehen.

3. Verfahren zur Herstellung von 1,3-Dichlor-2,2-dimethyl-propan-1,3-dicarbonsäureestern der Formel II (oben), dadurch gekennzeichnet, daß man 2,2-Dimethyl-propan-1,3-dicarbonsäure-derivate der Formel III 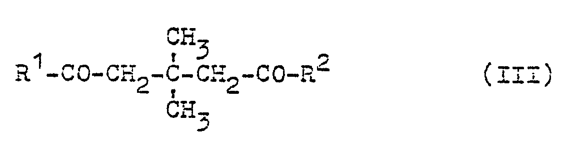 in welcher
R¹ für Hydroxy oder Alkoxy steht und
R² für Hydroxy oder zusammen mit R' für ein Anhydrid-Sauerstoffatom steht,
mit einem Säurechlcrid, gegebenenfalls in Gegenwart eines Katalysators, und mit einem Chlorierungsmittel bei Temperaturen zwischen 0 und 15o°C umsetzt und die auf diese Weise gebildeten 1,3-Dichlor-2,2-dimethylpropan-l,3-dicarbonsäure-derivate der Formel IV 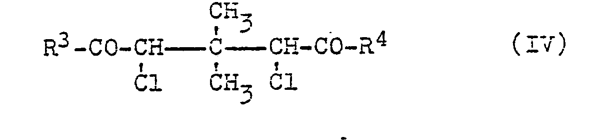 in welcher
R³ für Chlor oder Alkoxy steht und
R⁴ für Chlor oder zusammen mit R³ für ein Anhydrid-Sauerstoffatom steht,
durch Umsetzung mit Alkoholen nach üblichen Methoden verestert.

1. 1,3-Dichlor-2,2-dimethyl-propan-1,3-dicarbonsäure- derivate der Formel IV 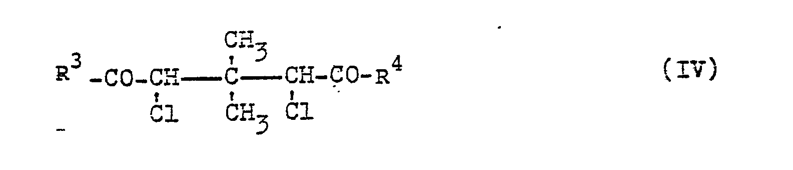 in welcher
_{R}³ für Chlor oder Alkoxy steht und
R⁴ für Chlor oder zusammen mit R³ für ein Anhydrid-Sauerstoffatom steht.
